# EUROPEAN PATENT APPLICATION

(11) **EP 3 566 751 A1**
(43) Date of publication of application: **13.11.2019**
(21) Application number: 19181993.7
(22) Date of filing: 18.02.2013
(51) Int. Cl.: A61Q 19/00, A61Q 5/00, A61Q 17/04, A61Q 1/02, A61Q 1/06, A61Q 5/12, A61Q 19/08, A61K 8/92, A61K 8/99, A61K 8/9789

(54) **COSMETIC COMPOSITIONS CONTAINING CLOUDBERRY CELL CULTURE PREPARATION**

(30) Priority: 21.02.2012 FI 20125200
(62) Divisional of application: 13751387.5
(71) Applicant: Lumene Oy, 02780 Espoo (FI)
(72) Inventor: BACKMAN, Josefin, 10300 Karis (FI); ISOHANNI, Tiina, 02400 Kirkkonummi (FI); OKSMAN-CALDENTEY, Kirsi-Marja, 02044 VTT (FI); NOHYNEK, Liisa, 02044 VTT (FI); RISCHER, Heiko, 02044 VTT (FI); PUUPPPONEN-PIMIÄ, Riitta, 02044 VTT (FI)
(74) Representative: Seppo Laine Oy

(57) **Abstract**

The present invention is directed to cosmetic compositions containing cloudberry (*Rubus chamaemorus*) cell culture preparation. The present invention is further directed to cosmetic compositions containing cloudberry cell culture preparation together with cloudberry seed oil and/or cloudberry seed extract. These components can be used, for example, in emulsion creams, foundation creams, skin serums, lipsticks, mascaras, hair cosmetics products, as well as in products for skin hygiene.

## Description

### FIELD OF THE INVENTION

The present invention is directed to cosmetic compositions containing cloudberry (*Rubus chamaemorus*) cell culture preparation. The present invention is further directed to cosmetic compositions containing cloudberry cell culture preparation together with cloudberry seed oil and/or cloudberry seed extract. These components can be used, for example, in emulsion creams, foundation creams, skin serums, lipsticks, mascaras and hair cosmetics products, as well as in products for skin hygiene.

### BACKGROUND OF THE INVENTION

Stem cells are found in all multicellular organisms. They can divide and differentiate into diverse specialized cell types and can also self-renew to produce more stem cells. In mammals, two types of stem cells exist: embryonic stem (ES) cells, which are isolated from the inner cell mass of blastocysts, and adult stem cells, which are found in various tissues.

The skincare world has recently become interested in utilizing stem cells in various skincare products and several companies have indeed been creating products with specialized peptides and enzymes or plant stem cells. The company called Proteonomix (earlier known as National Stem Cell), for example, has incorporated into their skin care product an enzyme secreted from human embryonic stem cells.

Plant callus is a mass of somatic cells that undergo dedifferentiation to give rise to totipotent embryogenic cells, which temporarily gain the ability to proliferate and/or regenerate an embryo. Callus and plant stem cells are fundamentally different from each other despite the fact that callus exhibits a number of stem cell-like properties for a temporary period and that it has been cultured for useful plant compounds as an alternative source of plant stem cell. Callus is similar to plant stem cell in its ability to differentiate, but the two are different in their origin. While plant stem cell exists in the meristematic tissues of plant, callus is obtained as a temporary response to cure wound in somatic cell. Only plant stem cells embedded in meristems can divide and give rise to cells that differentiate while giving rise to new stem cells. Cultured callus-derived cells provide a cost-effective, environmentally friendly, and sustainable source of important natural products.

Extracts from plant stem cells or dedifferentiated plant cells have been used in cosmetics. The Institute for Biotechnological Research (IRB) has released an anti-aging ingredient based on edelweiss stem cells. Edelweiss produces protective substances which the plant uses to defend itself against harsh climatic and environmental conditions. These substances may be used to protect skin. The company called XTEMcell Stem Cell SkinCare has come out with various skincare products containing cloned plant cells from the date palm from rare, 100% organic, nutrient-rich plants. In addition, US20110159121 (LifeSpan Extension, LCC) describes methods for identifying, producing, and using compositions, which can contain plant stem cells that can be derived from cloudberry meristematic cells. The compositions containing stem cells can be used in the form of cosmetic preparations. WO2010/067212 describes a cosmetic composition containing a complex of active vegetal stem cells (of *Malus domestica* or of *Buddleja davidii*) and Teprenone. In addition, EP1985280 describes a cosmetic product utilizing dedifferentiated plant cells for protecting and renewing skin stem cells. US20080299092 includes a cosmetic preparation for protecting stem cells against intrinsic and extrinsic stress factors. The preparation contains dedifferentiated plant cells from fruits of *Malus domestica* (apple) cultivar Uttwiler Spaetlauber. The cosmetic composition that is described in KR2009108812 e.g. for preventing skin aging and for improving skin-moisture contains apple stem cells together with Hibiscus seed oil extract as active ingredients.

Most plant oils contain unsaturated fatty acids, such as oleic, linoleic and linolenic acids to various degrees. Also the seeds of berries are known to contain oils, the major component of which are the triacylglycerols, which form an important energy reserve for germinating seeds. The fatty acid composition of seed oil triacylglycerols varies widely among plant species, and tends to be characteristic of particular plant families. The fatty acid composition of the seed oils of edible berries has been studied. One study included the analysis of 22 common edible berries including cloudberry (Johansson A. et al., Z. Lebensm. Unters. Forsch. A (1997) 204; 300-307). In this study the seeds were separated from the berries, dried and crushed, and the oil extracted with a mixture of chloroform and methanol, and the eluted neutral lipids analysed for their fatty acid composition. Solvent extraction methods are, however, not applicable for the preparation of substances intended for cosmetic use, due to an insufficient degree of purity and the inclusion of solvent residues. Manninen *et al.* (Manninen, P. et al., J. Agric. Food Chem. 1997, 45, 2533-2538) describe the use of supercritical fluid extraction for the extraction of oil from cloudberry seeds in order to determine its composition.

Various cloudberry-derived substances have been used in cosmetic compositions. EP1337224 (Lumene Oy and Aromtech Ltd) describes cosmetic compositions containing cloudberry seed oil. WO2009125071 (Aromtech Ltd) discloses extracts from berries, wherein the berries can be cloudberries. US20110033407 and EP2262473 describe hair treatment means with cloudberry extracts. WO2006117430 relates to use of composition comprising at least one triglyceride of at least one unsaturated fatty acid for manufacture of a product for skin lightening.

Currently there are numerous topical cosmetic preparations on the market aimed at improving the aesthetic aspect of the skin and the body and to mitigate the wrinkles of the face. Some of the cosmetic products incorporate components of plant origin, typically extracts or mature plant cells. The current preparations contribute mainly to increasing the hydration and the lipidic layer of the skin without, however, activating the deeper cellular processes which preside over the renewal of the epidermis and the dermis. The newest technology in skincare products is based on various plant stem cell derived biomaterials. Although the technology to produce plant stem cells is quite well known, the real challenge lies in successfully scaling up to produce commercially important quantities of plant stem cells and biomaterials derived from those.

### SUMMARY OF THE INVENTION

The present invention is directed to cosmetic compositions which contain cosmetically acceptable substances and, in addition, a cloudberry cell culture preparation, which is established from cloudberry callus cells. The invention further relates to use of such compositions as, e.g., day creams, foundation creams, lipsticks, skin serums, mascaras, products for hair and/or scalp care, washing products for skin or hair, or as products for skin hygiene. The compositions according to the invention may also comprise cloudberry seed oil and/or cloudberry seed extract.

### DETAILED DESCRIPTION OF THE INVENTION

The present inventors have found that cloudberry cell culture preparation which is used according to the present invention is especially advantageous for inclusion in cosmetic compositions. It was shown that cloudberry cell suspension has both antioxidant activity and anti-aging effect. It also protects human skin cells from UV radiation effects. Furthermore, cloudberry cell suspension was also shown to have increasing effect on the procollagen I synthesis of aged fibroblasts.

For the purposes of the present invention the term "cloudberry cell culture preparation" means a preparation, preferably in the form of freeze-dried powder, which is obtained by a method established by the scientists of VTT. (Nohynek, L., Bailey, M., Tähtiharju, J., Oksman-Caldentey, K.-M., Rischer, H. & Puupponen-Pimiä, R.; manuscript in preparation). In said method callus is first produced from sterile cuts of cloudberry plant. The material is maintained on a medium favouring continuous growth of the non-differentiated cells. Selected callus is then chosen for suspension culture, which is stepwise scaled up to large scale cultivation in a bioreactor. The biomass is harvested, washed with water and freeze-dried. The freeze-dried powder so obtained is very useful in cosmetic compositions of the present invention. However, it is possible to extract the powder with, e.g. methanol or ethanol. After filtering, such an extract can be used in cosmetic compositions of the invention instead of the freeze-dried powder.

Cloudberry seed oil and cloudberry seed extract, which may optionally be included in the cosmetic compositions of the present invention are produced as follows. Cloudberry seeds are first milled and extracted with CO₂. From this extraction the separated cloudberry seed oil is recovered, subjected to after-treatment and packed. The seed extract is obtained by extracting the residue from the CO₂ extraction process by using a solvent different from CO₂. It is possible to use water, ethanol, ethanol/water, acetone, butylene glycol, butylene glycol/water, glycerine or their mixtures as solvents in the extraction of the residual material. According to one preferred embodiment of the invention butylene glycol is used as a solvent. After the second extraction, the solids are separated from the extract. Before packaging the extract is subjected to microbial filtration.

The cloudberry cell culture preparation has a high protein content compared to cloudberry berry fruit, but contains about the same amount of fat as cloudberry berry fruit. Furthermore, the cloudberry cell culture preparation has rather high dietary fiber content, although no seeds are present. Flavanols are found in cloudberry cell culture in higher amounts than in berry fruit (ten times more). Also, the cloudberry cell culture preparation produces small amounts of ellagitannin compounds.

The cloudberry cell culture preparation as defined above has been found to have advantageous properties in skin care. Anti-aging effect was evaluated from ethanol extract of cloudberry cell suspension by detecting expression of senescence associated β-galactosidase and matrix metalloproteinase-1 in hydrogen peroxide induced senescence cells, i.e. fibroblasts and keratinocytes. The cloudberry cell culture extract prevented the cell senescence in both of these assays.

The antioxidant activity of cloudberry cell suspension was evaluated from methanol extracts of the suspension culture. Radical scavenging activity was expressed as Trolox TEAC value, which is the concentration of the studied sample solution, which is as antioxidative as Trolox (synthetic vitamin E analog).TEAC value for cloudberry cell suspension was 492.

Cloudberry cell culture preparation also defends human skin tissues from UV radiation effects. When the tissues were pre-treated and post-treated with the cloudberry cell culture extract, significantly fever apoptotic cells were detected and the number of cell layers was comparable to those of the control (without UV exposure).

In addition, cloudberry cell suspension was found to present increasing effect on procollagen I synthesis. To study the effect of cloudberry cell suspension on procollagen I synthesis, "aged" and normal fibroblasts were cultured. After incubation the medium was removed and replaced with culture medium containing cloudberry cell suspension or the reference. The cells were then further incubated, and the quantities of procollagen I in culture supernatants were measured. Cloudberry cell suspension presented a concentration-dependent increasing effect on procollagen I synthesis of "aged" fibroblasts.

Furthermore, gene expression changes after induced cell stress in the presence of cloudberry cell suspension or cloudberry seed extract were effected to show advantageous properties of these ingredients of the cosmetic composition of the invention.

Heat shock protein 70 (HSP70) is induced upon stress, including oxidative stress. Following stress-induced damage, HSP70 functions as a molecular chaperone to stabilize pre-existent proteins against aggregation. Upon oxidative stress cloudberry cell suspension and cloudberry seed extract was found to reduce the level of HSP70, indicating that the compounds have a protective effect against cell stress in keratinocytes when HSP70 is used as a marker gene. On the other hand, matrix metalloproteinase 13 (MMP13) is a key proteinase in degradation of type II collagen. Oxidative stress doubles the MMP13 expression. Cloudberry seed extract and cloudberry cell suspension was found to either reduce or maintain MMP13 levels similar to control cells upon oxidative stress, suggesting that these compounds decrease collagen degradation in the presence of oxidative stress when MMP13 is used as a marker gene.

The advantageous properties and effects of cloudberry seed oil are discussed in detail in EP patent No. 1 337 224 (Lumene Oy and Aromtech Ltd), which is incorporated herein by reference in its entirety.

The cloudberry seed extract, which can be prepared as described above may be purchased from Aromtech Ltd by trade name SUN ESSENCE HG. According to the information by Aromtech Ltd the SUN ESSENCE HG Cloudberry Seed Extract has been shown to impart a strong inhibition of elastase activity. When the elastase enzyme activity was measured at 2% active levels the reduction in elastase activity was 13.8 %, whereas the solvent vehicle did not have any effect. The results are supportive of the use of SUN ESSENCE HG as an anti-ageing active.Cloudberry seed extract contains a combination of flavonoids, tannins, polysaccharides and amino acids. The flavonoids are antioxidative compounds with many favourable health effects. Cloudberry seed extract is also rich in ellagitannins. Ellagitannins are hydrolysed to ellagic acid. Ellagic acid, a polyphenolic compound mostly found in berries and pomegranate, is shown to possess growth-inhibiting and apoptosis-promoting activities in cancer cells. Also, it has been shown that ellagic acid alleviates skin wrinkle and inflammation induced by UV-B irradiation (Bae et al., Exp. Dermatol., 2010, 19, e182-e190). As discussed above, the gene expression changes were found also in the presence of cloudberry seed extract. Thus, some of the advantageous and desired properties of those cosmetic compositions according to the invention which comprise cloudberry seed oil and cloudberry seed extract are derived from these ingredients.

The cosmetic composition according to the invention can be, for example, an emulsion cream, such as a day cream or a foundation cream; a lipstick; a skin serum or a hair cosmetics product, such as a composition for hair conditioning or a composition for scalp treatment.

The cosmetic composition according to the invention contains, depending on the intended use of the composition, cloudberry cell culture preparation in powder form in an amount of 0.001 to 25 % by weight, more preferably the amount of the powder is 0.01 to 5 % by weight, and most preferably 0.01-1 % by weight.

The cosmetic composition according to the invention may also contain, depending on the intended use of the composition, cloudberry seed oil in an amount of 0.1 to 25 % by weight, more preferably the seed oil content is 0.1 to 5 % by weight, and most preferably 0.5 to 3 % by weight. Alternatively, or in addition, the cosmetic composition may contain cloudberry seed extract in an amount of 0.1 to 25 % by weight, more preferably the seed extract content is 0.1 to 5 % by weight, and most preferably 0.5 to 2 % by weight.

Additionally, the cosmetic composition according to the invention may also contain, depending on the intended use of the composition, different caring agents which also include cloudberry seed oil. These caring agents are typically in the amount of 0.1 to 40 % by weight, more preferably the caring agent content of a product is 0.1 to 20 % by weight, and most preferably 1 to 5 % by weight.

The emulsion cream according to the invention containing cloudberry cell culture preparation, and optionally cloudberry seed oil and/or seed extract can be of the type oil-in-water emulsion, water-in-oil emulsion, water-oil-water emulsion or a microemulsion. The emulsion cream composition according to the invention contains cloudberry cell culture preparation in powder form, preferably 0.001 to 25 % by weight, more preferably 0.01 to 5 % by weight and most preferably 0.01 to 1 % by weight. The content of cloudberry seed oil in an emulsion cream may be in an amount of 0.1 to 25 % by weight. Preferably the seed oil content is 0.1 to 5 % by weight and most preferably 0.5 to 3 % by weight. In addition, the cosmetic composition may contain cloudberry seed extract in an amount of 0.1 to 25 % by weight. Preferably the seed extract content is 0.1 to 5 % by weight and most preferably 0.5 to 2 % by weight.

In the emulsion creams according to the invention the cloudberry cell culture preparation, seed oil and seed extract can be combined also with either synthetic or natural vitamins. The examples of these vitamins are retinol and A-vitamin palmitate. On the other hand, also different E-vitamins and E-vitamin derivatives, like tocopherol, C-vitamin and its derivatives, like ascorbyl palmitate and magnesium ascorbyl phosphate, panthenol and other B-vitamins and/or biotine may be used. The composition contains synthetic or natural vitamins preferably 0.01 to 10 % by weight, more preferably 0.02 to 5 % by weight.

The emulsion cream compositions according to the invention contain, in addition, one or more adjuvants acceptable in the field of cosmetics, such as preservation agents, thickening agents, moisturizing agents, and other suitable additives, such as for example perfumes and/or colouring agents. Suitable preservation agents are for example parabens, phenoxyethanol, imidazolidinyl urea and methyldibromo glutaronitrile. These preservation agents can be used alone or combined with each other. As thickening agent any thickening agent suitable in the field of cosmetics can be used, as long as it is compatible with the other components of the composition, for example xanthan gum and hydroxyethylcellulose, hydroxypropylmethylcellulose, Sclerotium gum, Chondrus Crispus, polyacrylates, polyacrylamides, cetearyl dimethicone crosspolymer and magnesium aluminium silicate. Thickening agents can be used alone or in combination with each other. Suitable moisturizing agents are for example heptyl undecylenate, hyaluronic acid or sodium PCA. In the emulsion creams according to the invention, also different skin conditioning agents, like for example tocopheryl acetate, and ethylhexylglycerin may be used.

In addition, one or more compounds acting as an emulsifier, i.e. a compound dispersing and stabilizing the oil in water is needed in the emulsion cream composition. Useful emulsifiers are all non-ionic emulsifiers accepted by the cosmetic legislation, such as, for example, glyceryl stearate, PEG-5 glyceryl stearate, PEG-1 00 stearate, PEG-30 dipolyhydroxystearate, lecithin, hydrogenated lecithine and PEG-8 bees wax, steareth-21, steareth-2, sorbitan olivate as well as a mixture of a fatty glucoside, such as for example cetearyl, cocoyl, or myristyl glucoside and a fatty alcohol, such as for example cetearyl, cetyl, stearyl, octyldodecanol, caprylic/capric triglyceride or myristyl alcohol. In addition, of anionic emulsifiers, for example stearic acid, sodium hydroxide and triethanolamine are useful. Also different chelating agents, like disodium EDTA and citric acid may be used.

The lipstick compositions according to the invention contain cloudberry cell culture preparation preferably 0.001 to 25 % by weight, more preferably 0.01 to 5 % by weight and most preferably 0.01 to 1 % by weight. In addition, these compositions may contain cloudberry seed oil in an amount of 0.01 to 25 % by weight. Preferably the seed oil content is 0.1 to 5 % by weight and most preferably 0.5 to 3 % by weight. In addition the cosmetic composition may contain cloudberry seed extract in an amount of 0.1 to 25 % by weight. Preferably the seed extract content is 0.5 to 2 % by weight.

The lipstick compositions according to the invention contain in addition to cloudberry cell culture preparation, cloudberry seed oil and seed extract also other substances, like different waxes, oils, colouring and pearlescent agents, which are acceptable in the field of cosmetics and which are traditional components of lipstick compositions. Usable waxes are the natural waxes, such as bees wax, candelilla, carnauba, cereal based waxes, jojoba wax and their derivatives, in addition paraffin waxes and synthetic polyethylenes. Lipstick compositions according to the invention can also contain emulsifiers like PEG-8, behenyl alcohol, arachidyl glucoside and arachidyl alcohol, thickening agents like ethylcellulose and various vitamins and derivates of those, like tocopherol, ascorbyl palmitate and ascorbic acid.

The lipstick compositions according to the invention can in addition contain one or more adjuvants and/or additives acceptable in the field of cosmetics, such as preservation agent. Suitable preservation agents are for example parabens and phenoxyethanol. These preservation agents can be used alone or in combination with each other.

The compositions according to the invention intended for hair or scalp care contain cloudberry cell culture preparation, preferably 0.001 to 10 % by weight, more preferably 0.01 to 5 % by weight and most preferably 0.01 to 1 % by weight. These compositions may also contain cloudberry seed oil in an amount of 0.01 to 25 % by weight. Preferably the seed oil content is 0.1 to 5 % by weight and most preferably 0.5 to 2 % by weight. In addition, these compositions may contain cloudberry seed extract in an amount of 0.1 to 25 % by weight. Preferably the seed extract content is 0.1 to 5 % by weight and most preferably 0.5 to 2 % by weight. Further, the compositions according to the invention intended for hair or scalp care may also contain different caring agents that include also cloudberry seed oil. These caring agents are typically in the amount of 0.1 to 40 % by weight. Preferably the caring agent content of a product is 0.1 to 20 % by weight.

The compositions according to the invention intended for hair or scalp care contain in addition to cloudberry cell culture preparation, cloudberry seed oil and seed extract also other substances acceptable in the field of cosmetics, like cationically active substances, such as cetrimonium chloride, in addition an emulsion forming substance, such as for example cetyl alcohol, cetearyl alcohol, ceteareth-20. In addition, the compositions intended for hair or scalp care can contain one or more adjuvants acceptable in the field of cosmetics, such as a cellulose derivative, ethanol and/or water. Also oils, waxes and fatty alcohols can be present in the compositions according to the invention intended for hair or scalp care.

The skin serums according to the invention have the molecular ability to penetrate the skin deep within the layers of the skin and deposit the nutrients where they will be needed.

The serum compositions according to the invention contain cloudberry cell culture preparation preferably 0.001 to 25 % by weight, more preferably 0.01 to 5 % by weight and most preferably 0.01 to 1 % by weight. The serum compositions may further contain cloudberry seed oil in an amount of 0.01 to 25 % by weight. Preferably the seed oil content is 0.1 to 5 % by weight and most preferably 0.5 to 3 % by weight. In addition the serum compositions may contain cloudberry seed extract in an amount of 0.1 to 25 % by weight. Preferably the seed extract content is 0.1 to 5 % by weight and most preferably 0.5 to 2 % by weight.

The serum compositions according to the invention typically contain also other substances acceptable in the field of cosmetics such as emulsifying agents, chelating agents, solvents, preservatives, stabilizers together with substances affecting the skin permeability of the composition. These substances may be e.g. methylpropanediol, glycerin, phenoxyethanol, hydroxyethyl acrylate/sodium acryloyldimethyl taurate copolymer, xanthan gum, propanediol, ammonium acryloyldimethyltaurate/VP copolymer, polyisobutene, disodium EDTA, lecithin, glucose, hydrogenated phosphatidylcholine, inulin lauryl carbamate, PEG-7 trimethylolpropane coconut ether, chondrus crispus, ethyl pyrrolidone, and cellulose gum. Additionally, the serum compositions according to the invention contain different moisturizing and skin conditioning agents such as heptyl undecylenate, ethylhexylglycerin, caprylyl glycol, ethylhexyl cocoate, peat extract and glycolipids.

As indicated, the cloudberry cell culture preparation is preferably used in the form of freeze-dried powder, and the amounts given above are calculated for said powder. However, in case methanol or ethanol extracts are used, it is within the expertise of a person skilled in the art to adapt the amounts correspondingly.

The invention is illustrated by means of the following examples.

### Example 1

### Method for producing cloudberry cell culture preparation in powder form

Cloudberry (*Rubus chamaemorus*) cell culture was established from wild cloudberry plant collected from a forest in southern Finland. Callus was produced from sterile cuts of the cloudberry plant, and maintained on a medium favouring continuous growth of the non-differentiated cells. Selected callus was chosen for suspension culture, which was stepwise scaled up to large scale cultivation in a bioreactor. The biomass was harvested, washed with water and freeze-dried. The powder so obtained was used in the compositions of examples 8 to 20 below.

### Producing ethanol extract from the cell culture preparation

The freeze-dried cloudberry cell culture was ground to fine powder and extracted with 70% EtOH (ethanol-water 70:30 vol/vol) using magnetic stirrer (250 rpm) overnight at room temperature. Extraction was repeated twice with 70% EtOH the next day, using sonication for 10 min, and pooled. Ethanol was evaporated from the filtered extracts with Rotavapor, and the extracts were freeze-dried and stored frozen.

### Example 2

### Evaluation of antioxidant activity

Methanol extract of cloudberry cell suspension culture cultivated in the bioreactor, as described in Example 1, was used to evaluate the antioxidant activity by 2,2-diphenyl-1-picrylhydrazyl (DPPH) radical scavenging method described by Malterud et al. (Pharmacology. Vol 47 (suppl. 1), 1993; 77-85). Radical scavenging activity was expressed as Trolox Equivalent Antioxidant Capacity (TEAC) value, which is the concentration of the studied sample solution (in g/L), which is as antioxidative as Trolox (synthetic vitamin E analog) in concentration 1g/L. Thus value 1 is equal to the value of Trolox, and values lower than 1 are more efficient compared to Trolox. TEAC value for cloudberry cell suspension was 492.

### Example 3

### Evaluation of anti-ageing effect

Anti-aging effect was evaluated from ethanol extract of cloudberry cell suspension by detecting expression of senescence associated β-galactosidase and matrix metalloproteinase-1 in hydrogen peroxide induced senescence cells (fibroblasts and keratinocytes). The cloudberry cell culture extract prevented the cell senescence in both of these assays.

### Example 4

### Protection from UV-radiation

To evaluate the UV radiation effects on human skin, reconstituted human skin (EpiDermFT tissues, MatTek) was utilized. The tissues were irradiated with UV for 24 h after pre-treatment with ethanol extract of cloudberry cell culture. After irradiation, the tissues were cultivated for another 24 h in the presence of the extract. Thereafter, they were fixed in paraformaldehyde and embedded in paraffin. The paraffin sections were stained with hematoxylin and eosin (HE) stain, and the histological examination was carried out. When the tissues were pre-treated and post-treated with the cloudberry cell culture extract, significantly fever apoptotic cells were detected and the number of cell layers was comparable to those of the control (without UV exposure).

### Example 5

### Effect on procollagen I synthesis

To study the effect of cloudberry cell suspension on procollagen I synthesis, "aged" (P17-F) and normal (P8-NHDF) fibroblasts were cultured. After 24 h of incubation the medium was removed and replaced with culture medium containing cloudberry cell suspension or the reference (TGF-β at 10ng/ml). The cells were then incubated for 72 h. After this, the quantities of procollagen I in culture supernatants were measured using ELISA kits according to the supplier's instructions. Cloudberry cell suspension presented a concentration dependent increasing effect on procollagen I synthesis of "aged" fibroblasts.

### Example 6

### Studying gene expression changes after induced cell stress in the presence of cloudberry cell suspension or cloudberry seed extract

Keratinocytes were incubated for 24 hours in the presence of the test compounds, whereafter the cells were exposed for 30 minutes to 500 µM H₂O₂ to induce oxidative stress. After washing off H₂O₂ the cells were further cultured for 6 hours to allow induction of gene expression. Following RNA isolation and preparation of cDNA, the messenger RNA levels of the selected genes were determined using Taqman PCR.

### (a) HSP70

Heat shock protein 70 (HSP70) is induced upon stress, including oxidative stress. Following stress-induced damage, HSP70 functions as a molecular chaperone to stabilize pre-existent proteins against aggregation. Upon oxidative stress cloudberry cell suspension and cloudberry seed extract reduced the level of HSP70, indicating that the compounds have a protective effect against cell stress in keratinocytes when HSP70 is used as a marker gene.

### (b) MMP13

Matrix metalloproteinase 13 (MMP13) is a key proteinase in degradation of type II collagen. Oxidative stress doubles the MMP13 expression. Cloudberry seed extract and cloudberry cell suspension either reduce or maintain MMP13 levels similar to control cells upon oxidative stress, suggesting that these compounds decrease collagen degradation in the presence of oxidative stress when MMP13 is used as a marker gene.

### Example 7

### Processing cloudberry seeds to cloudberry seed oil and cloudberry seed extract

In the present study, for cloudberry seed oil and seed extract production cleaned cloudberry seeds were first milled and extracted with CO₂. From this extraction the separated seed oil was recovered. The residue from a CO₂ extraction process was extracted by using butylene glycol. After the butylene glycol extraction the solids were separated from the extract. Before packaging the extract was subjected to microbial filtration.

### Example 8

An example of ingredients for a ready-for-use emulsion cream composition for facial and throat skin care containing cloudberry seed oil, cloudberry seed extract and cloudberry cell culture preparation powder is shown in the following Table 1. The described emulsion cream composition moisturizes, brightens and softens the skin. In addition the emulsion cream according to the invention possesses antioxidant properties.

**Table 1. Day Cream.**

| INGREDIENTS | Ratio % |
|---|---|
| Aqua (Water) | Add 100 |
| Steareth-2 | 4.0-5.0 |
| Dimethicone | 3.0-5.0 |
| Glycerin | 2.0-5.0 |
| Steareth-21 | 2.500 |
| Octyldodecanol | 1.0-3.0 |
| Stearic Acid | 1.0-3.0 |
| Cloudberry Seed Oil | 0.1-5.0 |
| Cloudberry Seed Extract | 0.1-5.0 |
| Cloudberry Cell Culture Preparation | 0.01-5.0 |
| Cetearyl Alcohol | 1.500 |
| Heptyl Undecylenate | 1.0-2.0 |
| Caprylic/Capric Triglyceride | 1.000 |
| PEG-30 Dipolyhydroxystearate | 0.5-1.0 |
| Aluminum Starch Octenyl Succinate | 0.5-1.0 |
| Phenoxyethanol | 0.5-1.0 |
| Cetearyl Dimethicone Crosspolymer | 0.5-1.0 |
| Tocopheryl Acetate | 0.2-0.8 |
| PPG-15 Stearyl Ether | 0.3-0.6 |
| Parfum (Fragrance) | 0.390 |
| Lecithin | 0.203 |
| Xanthan Gum | 0.15-0.2 |
| Acrylates/C10-30 Alkyl Acrylate Crosspolymer | 0.100 |
| Disodium EDTA | 0.100 |
| Magnesium Ascorbyl Phosphate | 0.100 |

### Example 9

An example of ingredients for ready-for-use emulsion cream composition containing cloudberry cell culture preparation powder for facial and throat skin care is presented in the following Table 2. The emulsion cream composition improves the revitalization of the skin. In addition, it reduces, softens and smoothes fine lines in the skin. It has also antioxidant properties.

**Table 2. Day Cream.**

| INGREDIENTS | Ratio % |
|---|---|
| Aqua (Water) | Add 100 |
| Steareth-2 | 4.0-5.0 |
| Dimethicone | 3.0-5.0 |
| Glycerin | 2.0-5.0 |
| Steareth-21 | 2.500 |
| Octyldodecanol | 1.0-3.0 |
| Stearic Acid | 1.0-3.0 |
| Cloudberry Cell Culture Preparation | 0.01-5.0 |
| Cetearyl Alcohol | 1.500 |
| Heptyl Undecylenate | 1.0-2.0 |
| Caprylic/Capric Triglyceride | 1.000 |
| PEG-30 Dipolyhydroxystearate | 0.5-1.0 |
| Aluminum Starch Octenyl Succinate | 0.5-1.0 |
| Phenoxyethanol | 0.5-1.0 |
| Cetearyl Dimethicone Crosspolymer | 0.5-1.0 |
| Tocopheryl Acetate | 0.2-0.8 |
| PPG-15 Stearyl Ether | 0.3-0.6 |
| Parfum (Fragrance) | 0.390 |
| Lecithin | 0.203 |
| Xanthan Gum | 0.15-0.2 |
| Acrylates/C 10-30 Alkyl Acrylate Crosspolymer | 0.100 |
| Disodium EDTA | 0.100 |
| Magnesium Ascorbyl Phosphate | 0.100 |

### Example 10

An example of ingredients for a foundation cream composition containing cloudberry cell culture preparation powder for facial skin is presented in Table 3. The cream moisturizes the skin, and protects it from UV-radiation. The cream also gives the skin light coverage and colour.

**Table 3. Foundation Cream**

| INGREDIENTS | Ratio % |
|---|---|
| Aqua (Water) | 50.0-55.0 |
| Cyclopentasiloxane | 10.0-15.0 |
| (CI 77891) Titanium Dioxide | 5.0-10.0 |
| Cyclohexasiloxane | 3.0-5.0 |
| Isododecane | 2.0-10.0 |
| Ethylhexyl Methoxycinnamate (Octinoxate) | 1.0-6.0 |
| Polyglyceryl-4 isostearate | 1.0-2.0 |
| Magnesium Sulfate | 1.0-2.0 |
| Cetyl PEG/PPG-10/1 Dimethicone | 1.0-2.0 |
| Hexyl laurate | 1.0-2.0 |
| (CI 77491) Iron Oxides | 1.0-2.0 |
| MICA | 1.0-2.0 |
| Nylon-12 | 0.5-1.0 |
| Propylene Glycol | 0.5-1.0 |
| Titanium Dioxide | 0.5-1.0 |
| Phenoxyethanol | 0.5-1.0 |
| Disteardimonium Hectorite | 0.5-1.0 |
| Silica | 0.5-1.0 |
| (CI 77492) Iron Oxides | 0.1-1.0 |
| Butylene Glycol | 0.1-1.0 |
| Cloudberry Cell Culture Preparation | 0.01-5.0 |
| (CI 77499) Iron Oxides | 0.1-1.0 |
| Perfluorooctyl Triethoxysilane | 0.1-1.0 |
| Propylene Carbonate | 0.05-0.3 |
| Dimethicone Crosspolymer | 0.05-0.3 |
| Methicone | 0.05-0.3 |
| Parfum (Fragrance) | 0.05-0.3 |
| Ethylhexylglycerin | 0.05-0.3 |
| Alumina | 0.05-0.3 |
| Triethoxycaprylylsilane | 0.05-0.3 |
| | 100.001 |

### Example 11

An example of ingredients for a lipstick composition containing cloudberry cell culture preparation powder is presented in Table 4. The lipstick composition according to the invention moisturizes the lips and provides lip care.

**Table 4. A lipstick composition.**

| INGREDIENTS | Ratio % |
|---|---|
| Ricinus communis Seed Oil | Add 100 |
| Squalane | 17.500 |
| Candelilla cera | 12.100 |
| Apricot kernel oil peg-6 esters | 8.000 |
| Acetylated lanolin | 5.900 |
| Aqua | 5.000 |
| Peg-8 beeswax | 4.700 |
| Hydrogenated polydecene | 4.000 |
| Butyrospermum parkii | 4.000 |
| Isopropyl myristate | 3.900 |
| Cera alba | 2.300 |
| Ethylcellulose | 2.000 |
| Cloudberry Cell Culture Preparation | 0.01-2.0 |
| Polyethylene | 2.000 |
| Mica | 1.800 |
| Arachidyl alcohol | 1.400 |
| Behenyl alcohol | 1.300 |
| Arachidyl glucoside | 1.300 |
| Perfume | 1.000 |
| Cloudberry Seed oil | 1.000 |
| Carnauba Wax | 0.900 |
| Propylparaben | 0.100 |
| PEG-8 | 0.100 |
| Tocopherol | 0.035 |
| Ascorbyl palmitate | 0.006 |
| Ascorbic Acid | 0.001 |
| Citric Acid | 0.001 |
| Colours | 4.800 |

### Example 12

Another example of a lipstick composition containing cloudberry cell culture preparation powder is presented in Table 5.

**Table 5. Another lipstick composition containing cloudberry cell culture preparation powder.**

| INGREDIENTS | Ratio % |
|---|---|
| Ricinus communis Seed Oil | Add 100 |
| Oleyl alcohol | 20.000 |
| Candelilla cera | 9.800 |
| Cera alba | 5.700 |
| Acetylated lanolin | 4.700 |
| Isopropyl lanolate | 4.000 |
| Isopropyl myristate | 3.200 |
| Hydrogenated polydecene | 3.000 |
| Ethylhexyl methoxycinnamate | 2.000 |
| Mica | 1.800 |
| Microcrystalline wax | 1.600 |
| Cloudberry Seed oil | 1.000 |
| Cloudberry Cell Culture Preparation | 0.01-2.0 |
| Perfume | 1.000 |
| Carnauba | 0.700 |
| Propyl paraben | 0.090 |
| PEG-8 | 0.040 |
| Tocopherol | 0.020 |
| Ascorbyl palmitate | 0.003 |
| Ascorbic Acid | 0.001 |
| Citric Acid | 0.001 |
| Colours | 4.800 |

### Example 13

An example of ingredients for a lipstick composition containing cloudberry cell culture preparation powder together with cloudberry seed oil and seed extract is presented in Table 6. The lipstick composition according to the invention has a lip protecting and caring effect.

**Table 6. A lipstick composition containing cloudberry seed oil and cloudberry seed extract together with cloudberry cell culture preparation powder.**

| INGREDIENTS | Ratio % |
|---|---|
| Ricinus communis Seed Oil | Add 100 |
| Squalane | 17.500 |
| Candelilla cera | 12.100 |
| Apricot kernel oil peg-6 esters | 8.000 |
| Acetylated lanolin | 5.900 |
| Aqua | 5.000 |
| Peg-8 beeswax | 4.700 |
| Hydrogenated polydecene | 4.000 |
| Butyrospermum parkii | 4.000 |
| Isopropyl myristate | 3.900 |
| Cera alba | 2.300 |
| Ethylcellulose | 2.000 |
| Cloudberry Seed Oil | 0.1-5.0 |
| Cloudberry Seed Extract | 0.1-5.0 |
| Cloudberry Cell Culture Preparation | 0.01-2.0 |
| Polyethylene | 2.000 |
| Mica | 1.800 |
| Arachidyl alcohol | 1.400 |
| Behenyl alcohol | 1.300 |
| Arachidyl glucoside | 1.300 |
| Perfume | 1.000 |
| Carnauba Wax | 0.900 |
| Propylparaben | 0.100 |
| PEG-8 | 0.100 |
| Tocopherol | 0.035 |
| Ascorbyl palmitate | 0.006 |
| Ascorbic Acid | 0.001 |
| Citric Acid | 0.001 |
| Colours | 4.800 |

### Example 14

Another example of a lipstick composition containing cloudberry cell culture preparation powder, cloudberry seed oil and seed extract is presented in Table 7.

**Table 7. Another lipstick composition containing cloudberry seed oil, cloudberry seed extract and cloudberry cell culture preparation powder.**

| INGREDIENTS | Ratio % |
|---|---|
| Ricinus communis Seed Oil | Add 100 |
| Oleyl alcohol | 20.000 |
| Candelilla cera | 9.800 |
| Cera alba | 5.700 |
| Acetylated lanolin | 4.700 |
| Isopropyl lanolate | 4.000 |
| Isopropyl myristate | 3.200 |
| Hydrogenated polydecene | 3.000 |
| Ethylhexyl methoxycinnamate | 2.000 |
| Cloudberry Seed Oil | 0.1-5.0 |
| Cloudberry Seed Extract | 0.1-5.0 |
| Cloudberry Cell Culture Preparation | 0.01-2.0 |
| Mica | 1.800 |
| Microcrystalline wax | 1.600 |
| Perfume | 1.000 |
| Carnauba wax | 0.700 |
| Propyl paraben | 0.090 |
| PEG-8 | 0.040 |
| Tocopherol | 0.020 |
| Ascorbyl palmitate | 0.003 |
| Ascorbic Acid | 0.001 |
| Citric Acid | 0.001 |
| Colours | 4.800 |

### Example 15

An example of ingredients for a serum product containing cloudberry cell culture preparation powder is presented in Table 8.

**Table 8. A serum composition.**

| INGREDIENTS | Ratio % |
|---|---|
| Aqua (Water) | Add 100 |
| Heptyl Undecylenate | 4.000 |
| Methylpropanediol | 3.000 |
| Glycerin | 3.000 |
| Cloudberry Cell Culture Preparation | 0.01-2.0 |
| Phenoxyethanol | 1.000 |
| Hydroxyethyl Acrylate/Sodium Acryloldimethyl Taurate Copolymer | 0.300 |
| Xantan Gum | 0.1-0.4 |
| PVP | 0.200 |
| Ethylhexylglycerin | 0.200 |
| Propanediol | 0.163 |
| Ammonium Acryloyldimethyltaurate/ VP Copolymer | 0.150 |
| Caprylyl Glycol | 0.150 |
| Polyisobutene | 0.150 |
| Ethylhexyl Cocoate | 0.100 |
| Disodium EDTA | 0.100 |
| Lecithin | 0.056 |
| PEG-7 Trimethylolpropane Coconut Ether | 0.025 |
| Glucose | 0.008 |
| Peat Extract | 0.007 |
| Chondrus Crispus (Carrageenan) | 0.002 |
| Glycolipids | 0.000 |

### Example 16

An example of ingredients for a serum product containing cloudberry seed oil and seed extract in addition to cloudberry cell culture preparation powder is shown in Table 9.

**Table 9. A serum composition containing cloudberry seed oil, cloudberry seed extract and cloudberry cell culture preparation powder.**

| INGREDIENTS | Ratio % |
|---|---|
| Aqua (Water) | Add 100 |
| Heptyl Undecylenate | 4.000 |
| Methylpropanediol | 3.000 |
| Glycerin | 3.000 |
| Phenoxyethanol | 1.000 |
| Cloudberry Seed Oil | 0.1-5.0 |
| Cloudberry Seed Extract | 0.1-5.0 |
| Cloudberry Cell Culture Preparation | 0.01-2.0 |
| Hydroxyethyl Acrylate/Sodium Acryloldimethyl Taurate Copolymer | 0.300 |
| Xantan Gum | 0.266 |
| PVP | 0.200 |
| Ethylhexylglycerin | 0.200 |
| Propanediol | 0.163 |
| Ammonium Acryloyldimethyltaurate/ VP Copolymer | 0.150 |
| Caprylyl Glycol | 0.150 |
| Polyisobutene | 0.150 |
| Ethylhexyl Cocoate | 0.100 |
| Disodium EDTA | 0.100 |
| Lecithin | 0.056 |
| PEG-7 Trimethylolpropane Coconut Ether | 0.025 |
| Glucose | 0.008 |
| Peat Extract | 0.007 |
| Chondrus Crispus (Carrageenan) | 0.002 |

### Example 17

An example of a caring or conditioning product for hair. The composition containing cloudberry cell culture preparation powder is shown in Table 10. Cloudberry seed oil can be used in caring products to impart gloss to the hair and to improve the manageability of the hair.

**Table 10. A caring or conditioning product composition containing cloudberry cell culture preparation powder.**

| INGREDIENTS | Ratio % |
|---|---|
| Aqua (Water) | Add 100 |
| Cloudberry Cell Culture Preparation | 0.01-2.0 |
| Emulsifier, e.g. ceteareth 20 | 0.1-10 |
| Oils, waxes and/or fatty alcohols | 0.1-10 |
| Thickeners, e.g. cellulose derivatives | 0.1-10 |
| Cationic surfactants, e.g. cetrimonium chloride | 0.1-15 |
| Caring oils (e.g. Cloudberry Seed Oil) | 0.1-20 |
| Moisturizers, e.g. sorbitol | 0.1-10 |
| Polymers | 0.1-10 |
| Preservation agent, pH regulating agents, Perfume, Colour | |

### Example 18

Another example of a caring or conditioning product for hair. The composition containing cloudberry seed oil and seed extract in addition to the cloudberry cell culture preparation powder is shown in Table 11. Cloudberry seed oil can be used in caring products to impart gloss to the hair and to improve the manageability of the hair.

**Table 11. A caring or conditioning product composition containing cloudberry seed oil, seed extract, and cell culture preparation powder.**

| INGREDIENTS | Ratio % |
|---|---|
| Aqua (Water) | Add 100 |
| Cloudberry Seed Oil | 0.1-5.0 |
| Cloudberry Seed Extract | 0.1-5.0 |
| Cloudberry Cell Culture Preparation | 0.01-2.0 |
| Emulsifier, e.g. ceteareth 20 | 0.1-10 |
| Oils, waxes and/or fatty alcohols | 0.1-10 |
| Thickeners, e.g. cellulose derivatives | 0.1-10 |
| Cationic surfactants, e.g. cetrimonium chloride | 0.1-15 |
| Caring oils (e.g. Cloudberry Seed Oil) | 0.1-20 |
| Moisturizers, e.g. sorbitol | 0.1-10 |
| Polymers | 0.1-10 |
| Preservation agent, pH regulating agents, Perfume, Colour | |

### Example 19

An example of a caring product for scalp treatment. The composition containing cloudberry cell culture preparation powder is shown in Table 12. Products intended for special care are emulsions or creams which impart a caring and moisturizing effect to the scalp by means of the cloudberry seed oil.

**Table 12. A caring product composition for scalp treatment containing cloudberry cell culture preparation.**

| INGREDIENTS | Ratio % |
|---|---|
| Aqua (Water) | Add 100 |
| Ethanol | 10-60 |
| Cloudberry Cell Culture Preparation | 0.01-2.0 |
| Moisturizing agents, e.g. sorbitol | 0.1-10 |
| Caring agents, e.g. Cloudberry Seed Oil | 0.1-20 |
| Preservation agent, pH regulating agents, Colour, Perfume | |

### Example 20

Another example of a caring product for scalp treatment. The composition containing cloudberry seed oil and seed extract in combination with cloudberry cell culture preparation powder is shown in Table 13.

**Table 13. A caring product composition for scalp treatment containing cloudberry seed oil and seed extract in combination with cloudberry cell culture preparation powder.**

| INGREDIENTS | Ratio % |
|---|---|
| Aqua (Water) | Add 100 |
| Ethanol | 10-60 |
| Cloudberry Seed oil | 0.1-5.0 |
| Cloudberry Seed Extract | 0.1-5.0 |
| Cloudberry Cell Culture Preparation | 0.01-2.0 |
| Moisturizing agents, e.g. sorbitol | 0.1-10 |
| Caring agents, e.g. Cloudberry Seed Oil | 0.1-20 |
| Preservation agent, pH regulating agents, Colour, Perfume | |

The following embodiments are characteristic of the present invention:
1. A cosmetic composition which contains cosmetically acceptable substances, wherein the cosmetic composition in addition contains cloudberry cell culture preparation.
2. The cosmetic composition of embodiment 1, wherein the cloudberry cell culture preparation is in the form of freeze-dried powder derived from cloudberry callus cells.
3. The cosmetic composition of embodiment 1 or 2, wherein the amount of the cloudberry cell culture preparation is 0.001 to 25 % by weight of the composition.
4. The cosmetic composition of embodiment 3, wherein the amount of the cloudberry cell culture preparation is 0.01 to 1 % by weight of the composition.
5. The cosmetic composition according to any one of embodiments 1 to 4, wherein it in addition contains cloudberry seed extract and/or cloudberry seed oil.
6. The cosmetic composition of embodiment 5, wherein the amount of cloudberry seed extract is 0.1 to 5 % by weight of the composition and the amount of cloudberry seed oil is 0.1 to 5 % by weight of the composition.
7. The cosmetic composition of any one of embodiments 1 to 6, wherein it is a foundation cream or an emulsion cream composition.
8. The cosmetic composition of any one of embodiments 1 to 6, wherein it is a lipstick or a skin serum composition, or a composition for hair and/or scalp care.
9. Use of a composition according to any one of embodiments 1 to 6 as emulsion cream, foundation cream, lipstick or skin serum, or a product for hair and/or scalp care.

## Claims

1. A cosmetic composition which contains cosmetically acceptable substances, **characterized in that** it contains cloudberry cell culture preparation derived from cloudberry callus cells, wherein the amount of the cloudberry cell culture preparation is 0.001 to 25 % by weight of the composition.

2. The cosmetic composition according to claim 1, **characterized in that** the cloudberry cell culture preparation is in the form of freeze-dried powder.

3. The cosmetic composition according to claim 1 or 2, **characterized in that** the amount of the cloudberry cell culture preparation is 0.01 to 5 %, preferably 0.01 to 1 %, by weight of the composition.

4. The cosmetic composition according to any one of claims 1 to 3, **characterized in that** it in addition contains cloudberry seed extract and/or cloudberry seed oil, wherein the cloudberry seed extract and/or cloudberry seed oil have been obtained by
- milling cloudberry seeds and extracting with CO₂ to obtain cloudberry seed oil;
- extracting the residue from the CO₂ extraction by using a solvent different from CO₂ to obtain cloudberry seed extract.

5. The cosmetic composition according to claim 3 or 4, **characterized in that** the amount of cloudberry seed extract is 0.1 to 5 % by weight of the composition and the amount of cloudberry seed oil is 0.1 to 5 % by weight of the composition.

6. The cosmetic composition according to claim 4 or 5, **characterized in that** the cloudberry seed extract contains flavonoids, tannins, polysaccharides, amino acids, and is rich in ellagitannins.

7. The cosmetic composition according to any one of claims 1 to 6, **characterized in that** it is a foundation cream or an emulsion cream composition.

8. The cosmetic composition according to any one of claims 1 to 6, **characterized in that** it is a lipstick or a skin serum composition, or a composition for hair and/or scalp care.

9. Use of a composition according to any one of claims 1 to 6 as emulsion cream, foundation cream, lipstick or skin serum, or a product for hair and/or scalp care.
